# EUROPEAN PATENT APPLICATION

(11) **EP 1 790 681 A1**
(43) Date of publication of application: **30.05.2007**
(21) Application number: 05111203.5
(22) Date of filing: 24.11.2005
(51) Int. Cl.: C08J 9/00, B32B 5/20, B29C 44/00

(54) **A process for manufacturing a packaged sponge and packaged sponge thereby obtained**

(71) Applicant: Martini S.p.A., 43058 Coenzo di Sorbolo (Parma) (IT)
(72) Inventor: MARTINI, Fulvio, 43058, Coenzo di Sorbolo (PR) (IT)
(74) Representative: Giambrocono, Alfonso

(57) **Abstract**

The process for manufacturing a packaged sponge (1) comprises the steps of mixing a plastic material with a reagent to form a mixture (30), pouring the mixture (30) into a container (2), leaving the mixture (30) to cure to increase its volume and form a spongy mass (3), then closing the container (2) to produce said packaged sponge (1) to be forwarded to marketing. The packaged sponge (1) comprises the container (2) in which a spongy mass (3) is inserted. Each portion of the outer surface of the spongy mass (3) is in contact with that portion of the container (2) which faces it.

## Description

The present invention relates to a process for manufacturing a packaged sponge and a packaged sponge thereby obtained.

Reference will be made hereinafter to sponges of the type used for personal hygiene, for article cleaning, and in a medical environment.

Reference will also be made to sponges which, because of their particular combination of shapes and colours, are usable as toys, in particular for children; these can be, for example, sponges carrying comic figures, sponges for use as toys such as balls, or other articles in general.

Known sponges are currently produced as a large mass, for example as cubes having side dimensions of 1 or 2 metres. These cubic masses have their outer walls (which come into contact with the moulds) covered by an impermeable layer which renders them unsuitable for hygiene or cleaning use.

Consequently, such masses are firstly subjected to a cutting process by which the impermeable outer layer is removed, and which then cuts them into the desired shape, to produce the finished sponges.

After the sponges have been formed they are packaged, usually in bags containing one or more sponges.

This procedure is however extremely lengthy and complex, and requires a large number of very costly machines which are difficult to operate; for example, machines must be available for handling the cubic masses (which are very bulky and heavy), together with other machines for cutting the cubic masses into the finished sponges.

Moreover, this procedure does not enable sponges produced in this manner to be used for any of those uses which require the sponge to have been produced in a hygienic and/or aseptic and/or sterile environment.

A further drawback is that when the cubic mass is cut, considerable machining scrap is produced which, in addition to not being usable for production, has to be disposed of; this is very costly and results in considerable environmental impact.

The technical aim of the present invention is therefore to provide a process for manufacturing a packaged sponge and a packaged sponge thereby obtained by which the stated technical drawbacks of the known art are eliminated.

Within the scope of this technical aim, an object of the invention is to enable a packaged sponge to be manufactured rapidly and easily; in particular, the process of the invention does not require the use of a large number of costly machines of difficult operation for its implementation.

Another object of the invention is to provide a process by which production scrap is drastically limited; this results in a considerable saving in material costs because virtually all the material used in manufacture contributes to producing the final product, and a drastic reduction in disposal costs because there is no longer the need to dispose of material cut off in forming the finished sponges.

Moreover the environmental impact of the process of the invention is extremely small because the quantity of production scrap to be disposed of is very limited.

A further object of the invention is to manufacture sponges of any shape, including very complex, without particular process complications.

The technical aim, together with these and further objects, are attained according to the present invention by a process for manufacturing packaged sponges, characterised by mixing a plastic material with a reagent to form a mixture, pouring the mixture into a container, leaving the mixture to cure to increase its volume and form a spongy mass, then closing the container to produce said packaged sponge to be forwarded to marketing.

The present invention also relates to a packaged sponge comprising a container in which at least one spongy mass is inserted, characterised in that each portion of the outer surface of the spongy mass is in contact with that portion of the container which faces it.

Further characteristics of the present invention are defined in the other claims.

Further characteristics and advantages of the invention will be more apparent from the description of a preferred but non-exclusive embodiment of the process for manufacturing a packaged sponge and the packaged sponge thereby obtained, illustrated by way of non-limiting example in the accompanying drawings, in which:
Figure 1 is a perspective view of a packaged sponge according to the present invention;
Figure 2 is a perspective view of the packaged sponge of Figure 1, in which a closure film for a container containing the sponge is partially open;
Figure 3 is an exploded view of the packaged sponge of Figure 1;
Figure 4 is a cross-section through the packaged sponge of Figure 1; and
Figure 5 is a schematic view of a plant for implementing a process according to the present invention.

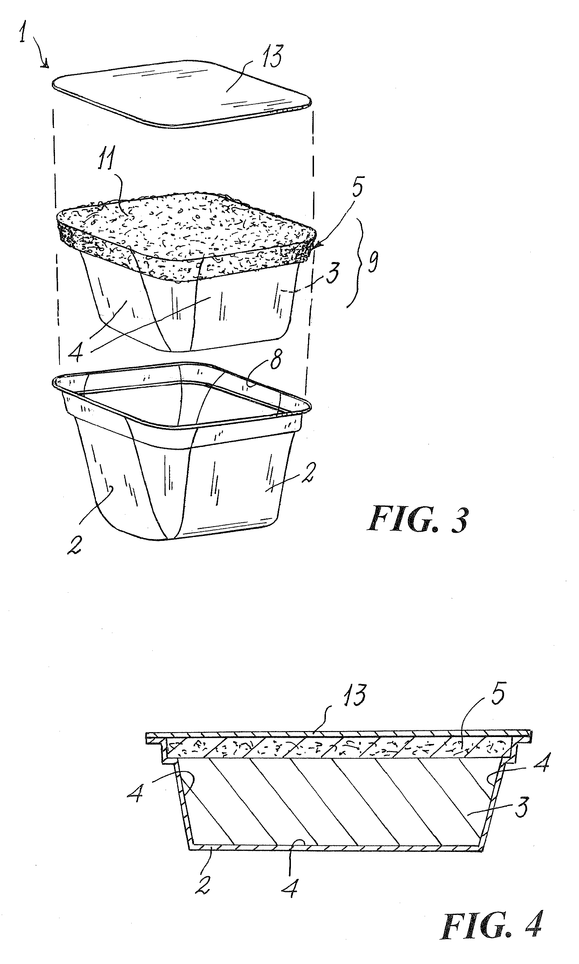

Said figures show a packaged sponge, indicated overall by the reference numeral 1. The packaged sponge 1 comprises a container 2 into which a spongy mass 3 is inserted; advantageously each portion of the outer surface 4 of the spongy mass 3 is in contact with that portion of the container 2 which faces it (Figure 4).

In practice, the spongy mass 3 is inserted into the container 2 without air bubbles or spaces filled with air being present between said spongy mass 3 and the container 2.

The container 2 is substantially rigid, i.e. is able to maintain its shape even when a liquid material is poured into its interior; it is made for example of a plastic material such as polypropylene or polyethylene or other material which enables the spongy mass to be detached therefrom; in other embodiments it is made of any material, lined (in its lower part in contact with the spongy mass 3) with polypropylene or polyethylene or teflon or other material enabling the spongy mass to be detached.

As shown in the accompanying figures, the spongy mass 3 can be secured to an insert 5 to form a finished sponge 9; the insert 5 is preferably secured to a portion of the spongy mass 3 facing an aperture 8 in the container 2 (through which the finished sponge 9 is extracted).

In other embodiments, not show in the accompanying figures, the insert 5 is secured to the spongy mass 3 but is positioned on the bottom of the container 2; in other embodiments the insert 5 is positioned in the interior of the spongy mass (for example to provide it with particular mechanical characteristics).

It is clear that if the insert 5 is absent, the finished sponge 9 consists of the actual sponge 3 itself.

The finished sponge 9 presents two large opposing usable surfaces, a first surface being that 11 defined by the insert 5 and can be formed as desired by suitably choosing the material of the insert, the second (opposite the surface 11) being defined by the spongy mass 3.

In different embodiments, the inserts 5 consist of abrasive fibres, elements (of natural or synthetic fibre, of sponge, of polyurethane, of rubber, etc.) shaped to provide good massaging or cleaning properties, powders of different particle size, fabric, flock, etc.

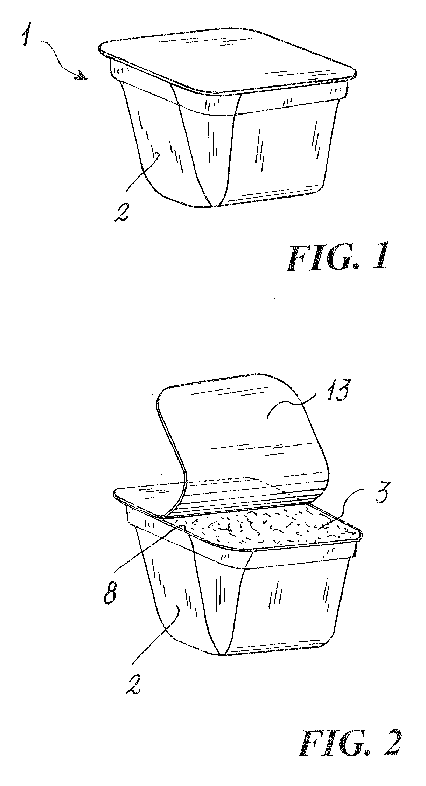

As shown in particular in Figures 2 and 3, the aperture 8 of the container 2 is suitably closed by a closure element, advantageously a film 13 which seals the container 2, or a rigid or semirigid cover formed of a material of usual type suitable for the purpose.

In this manner the finished sponge is completely protected by the container 2 and by the film 13 or cover element and can be easily stored and transported without the need for excessive care to prevent it being spoiled or damaged, for example by abrasion between several mutually contacting sponges. Moreover, that portion of the finished sponge 9 which faces the film 13 or closure element is in contact with the film 13 itself, thus ensuring both optimal use of the container (which is completely filled) and enabling the finished sponge 9 contained in a container to contribute to the support of any load lying on the container, so preventing this load from being supported only by the film 13; for example this configuration facilitates stacking of a plurality of packaged sponges 1 one on the others.

However, in other embodiments that portion of the finished sponge 9 which faces the film 13 is not in contact with said film 13.

Advantageously, the finished sponge 9 is sterile; in this respect, it is not touched by any operator and can be subjected to sterilization during the final step of its production process; the finished sponge, being isolated, is not contaminated by bacteria or other microorganisms which could alter its sterility, such as by contact with the outside environment.

The packaged sponge 1 of the invention is used very simply. The film 13 or closure element is firstly removed from the container 2, then the finished sponge 9 contained therein is removed.

The sponge can thus be used as a traditional sponge.

The present invention also relates to a process for manufacturing a packaged sponge.

The process consists of mixing a plastic material with a reagent to form a mixture; the mixture is poured into a container 2 for example of the tray type, and is left to cure to increase its volume.

The container 2 is then closed to form the packaged sponge 1, to be forwarded to marketing.

The plastic material preferably comprises a hydrophilic prepolymer in a percentage between 30-65% by weight on the mixture; the hydrophilic prepolymer used is for example the product marketed under the name Hypol by DOW.

Advantageously the hydrophilic prepolymers react with water (hence the product is particularly suitable for contact with the skin) under ambient (pressure and temperature) conditions to obtain a spongy mass without the impermeable outer layer which would hinder its use in hygiene and cleaning (an impermeable outer layer would have to be removed for this use).

The reagent consists of bacteriologically pure water, which is contained in the mixture in a percentage between 65-25% by weight.

The temperature at which the prepolymer and water are used can vary from 10°C to 45°C. Advantageously, surfactants are mixed into the mixture in a percentage less than 15% and preferably 2-10% by weight. In preferred embodiments, additives to provide the finished sponge 9 with predetermined characteristics are also mixed into the mixture.

Such additives comprise perfume and/or soap and/or agents able to soften the finished sponge 9, disinfectant, colouring, anti-oxidants, anti-microbics, vitamins, creams, oils, detergents, cosmetic and pharmacological substances, etc.

Again to provide the finished sponge 9 with particular characteristics, the insert 5 is positioned on the free surface of the mixture during curing, so that it remains secured to the spongy mass 3 when the curing process has terminated.

In practice, the insert 5 does not mix with the mixture (and hence is not contained also in the interior of the finished sponge) as do the additives, but forms one of the surfaces of the finished sponge 9.

In various embodiments of the finished sponge 9, the insert 5 consists of abrasive fibre, powders of different particle size, fabric, flock, elements shaped to provide massaging properties, etc.

In order to correctly secure the insert to the spongy mass 3 of the finished sponge 9 without this hindering the mixture curing, the insert 5 is positioned within 10 minutes from pouring.

The finished sponge 9 produced in this manner already presents optimal characteristics from the sterility and asepticity viewpoints as it has not been handled by an operator who could have transmitted germs to it, however in order to further guarantee this property (for example if the finished sponge 9 is to be used as a disinfectant vehicle), it can be sterilized after the container 2 has been closed.

Its closure is also hermetic, because of which its sterility, asepticity, cleanliness and protection cannot be compromised during its distribution and marketing.

Advantageously, that layer of the container 2 which comes into contact with the mixture is formed of polypropylene or polyethylene or teflon or other material enabling the spongy mass to be detached; this simplifies detachment of the spongy mass 3 from the container 2 when the finished sponge 9 is to be removed.

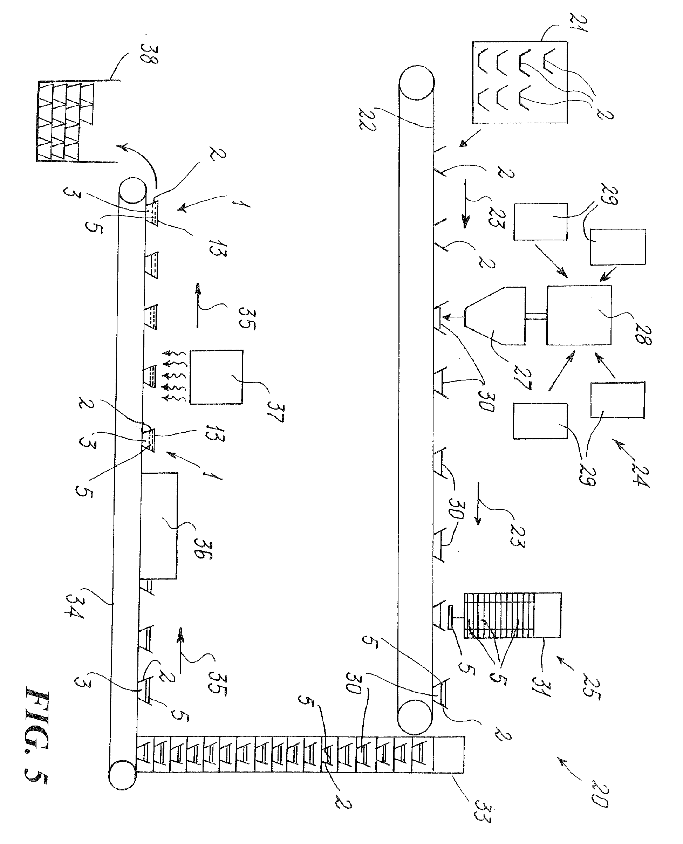

The process is implemented by a plant 20 of the type shown schematically in Figure 5.

The plant comprises a store 21 of containers 2; from the store 21 the containers 2 are passed in known manner onto a conveyor 22, for example of the belt type, which moves as indicated by the arrows 23.

The containers 2 are transported by the belt 22 below a filling station 24 and below a possible station for application of the insert 5.

The filling station comprises a pouring head 27 connected to a dynamic mixer 28 indicatively of capacity between 500 and 5000 grams per minute (in other embodiments a filling station 24 can be provided having several pouring heads 27 connected to a single mixer 28 or each connected to its own mixer 28; other configurations are also possible).

The mixer 28 is connected to one or more tanks 29 containing all the components required to prepare the mixture.

Figure 5 shows four tanks containing the hydrophilic prepolymer, the water, surfactants and the colouring.

When the container 2 is in the filling station 24 (i.e. when the container 2 resting on the conveyor 22 passes below the pouring head 27), mixture 30 is poured in; the quantity of mixture 30 poured in is between 5 and 100 grams depending on the final product and the container dimensions, to form a layer of liquid mixture on the base of the container 2.

After filling, the container 2 continues its movement and is conveyed by the conveyor 22 into the insert application station, where the insert 5 is applied for example by free fall from a store 31.

The time required to pass from the station 24 to the station 25 is that necessary for the process to take place correctly; to achieve this, the correct time requirement is predetermined by suitably setting the starts and stoppages of the conveyor 22, suitably adjusting the speed of the conveyor 22, providing a suitable length portion of the conveyor 22, etc.

The containers 2 (which contain the mixture 30 and the insert 5) are then housed in a vertical accumulator 33 having a capacity of 40 minutes of production; its purpose is to ensure curing of the mixture, which expands to assume the characteristics of a sponge (in addition to securing it to the insert).

The containers 2 are released onto a second belt conveyor 34 which moves as indicated by the arrows 35; at this point the containers contain the spongy mass 3 secured to the insert 5 (i.e. the finished sponge 9).

The containers 2 may pass through a liquid phase insertion station in which more or less viscous liquid is injected or otherwise inserted into the spongy mass. The containers 2 pass through a hot-sealing station 36 in which the container 2 is closed by the film 13, and finally through an ultraviolet or gamma ray sterilization station 37.

In a different embodiment (not shown in the figures), the sterilization station 37 precedes (in the direction of movement of the conveyor 34) the sealing station 36; hence in this embodiment the container 2 (with the finished sponge in its interior) is firstly sterilized and is then closed and sealed.

The packaged sponges hence formed are then housed in boxes 38 for their transport and marketing.

Modifications and variants, in addition to those already described, are possible, for example the insert application station 25 can be positioned upstream of the filling station 24, to form a packaged sponge 1 in which the insert 5 lies on the base of the container 2.

In a further embodiment, the insert application station 25 can be positioned between two filling stations 24, to insert the insert 5 into the interior of the spongy mass 3.

The process also enables articles of spongy material to be produced for use as gadgets or toys for children; for example dolls, balls, etc. can be easily and quickly produced.

In this case a very wide range of starting plastics can be used, as the sponge does not have to present an absorbing outer surface; in this respect, in this case starting plastic materials can be used (in the mixture) which, although reacting under conditions similar to those previously described, result in spongy masses presenting an impermeable outer layer (which would prevent their use in personal hygiene and/or article cleaning).

It has been found in practice that the process for manufacturing a packaged sponge and the packaged sponge thereby obtained according to the invention are particularly advantageous because they are very economical and present reduced environmental impact.

They also drastically limit production scrap, are very hygienic, are not allergic and present optimal characteristics for skin contact as catalysts are not used.

The process for manufacturing a packaged sponge and the packaged sponge thereby obtained are susceptible to numerous modifications and variants, all falling within the scope of the inventive concept; moreover all details can be replaced by technically equivalent elements.

In practice the materials used and their dimensions can be chosen at will in accordance with requirements and the state of the art.

## Claims

1. A process for manufacturing a packaged sponge (1), **characterised by** mixing a plastic material with a reagent to form a mixture (30), pouring the mixture (30) into a container (2), leaving the mixture (30) to cure to increase its volume and form a spongy mass (3), then closing the container (2) to produce said packaged sponge (1) to be forwarded to marketing.

2. A process as claimed in claim 1, **characterised in that** said plastic material comprises a hydrophilic polypropylene.

3. A process as claimed in the preceding claim, **characterised in that** said hydrophilic polypropylene is contained in a percentage between 30-65% by weight on the mixture.

4. A process as claimed in one or more of the preceding claims, **characterised in that** said reagent comprises water.

5. A process as claimed in the preceding claim, **characterised in that** the water is contained in the mixture in a percentage between 65-25% by weight.

6. A process as claimed in claims 2 and 4 or 2 and 5, **characterised in that** said water and said hydrophilic polypropylene have a temperature between 10-45°C.

7. A process as claimed in one or more of the preceding claims, **characterised by** mixing surfactants into the mixture in a pecentage less than 15% by weight, and preferably between 2-10%.

8. A process as claimed in one or more of the preceding claims, **characterised by** mixing additives into the mixture to confer predetermined characteristics on the finished sponge.

9. A process as claimed in one or more of the preceding claims, **characterised in that** during curing, an insert (5) is positioned on the free surface of the mixture, and remains secured to the sponge on termination of the curing process.

10. A process as claimed in one or more of the preceding claims, **characterised in that** said insert (5) is positioned within ten minutes from pouring.

11. A process as claimed in one or more of the preceding claims, **characterised by** sterilizing the finished sponge.

12. A process as claimed in one or more of the preceding claims, **characterised in that** the closure is hermetic.

13. A process as claimed in one or more of the preceding claims, **characterised in that** in said container (2), at least that layer which makes contact with the mixture is formed of polypropylene or polyethylene or teflon.

14. A process as claimed in one or more of the preceding claims, **characterised in that** said containers (2) pass through a liquid phase insertion station in which liquid is inserted into the spongy mass.

15. A packaged sponge (1) comprising a container (2) in which at least one spongy mass (3) is inserted, **characterised in that** each portion of the outer surface of the spongy mass (3) is in contact with that portion of the container (2) which faces it.

16. A packaged sponge (1) as claimed in the preceding claim, **characterised in that** said container (2) is substantially rigid.

17. A packaged sponge (1) as claimed in one or more of claims 15 onwards, **characterised by** comprising an insert (5) connected to said spongy mass (3) to form a finished sponge (9).

18. A packaged sponge (1) as claimed in one or more of claims 15 onwards, **characterised in that** said insert (5) is connected to a portion of the spongy mass (3) facing an aperture (8) of said container (2).

19. A packaged sponge (1) as claimed in one or more of claims 15 onwards, **characterised in that** said aperture (8) of said container (2) is closed by a closure element or a film (13).

20. A packaged sponge (1) as claimed in one or more of claims 15 onwards, **characterised in that** said film (13) or closure element seals said container (2).

21. A packaged sponge (1) as claimed in one or more of claims 15 onwards, **characterised in that** that portion of said finished sponge (9) which faces said film (13) or closure element is in contact with said film (13) or closure element.

22. A packaged sponge (1) as claimed in one or more of claims 15 onwards, **characterised in that** said finished sponge (9) is sterile.

23. A packaged sponge (1) as claimed in one or more of claims 15 onwards, **characterised in that** in said container (2), at least that layer in contact with the spongy mass (3) is formed of polypropylene or polyethylene or teflon.
